# EUROPEAN PATENT SPECIFICATION

(11) **EP 1 455 695 B1**
(45) Date of publication and mention of the grant of the patent: **13.06.2007**
(21) Application number: 02796922.9
(22) Date of filing: 18.12.2002
(51) Int. Cl.: A61F 5/44, A61F 5/453, B65B 69/00

(54) **FLUID COLLECTION APPARATUS**
VORRICHTUNG ZUM SAMMELN VON FLÜSSIGKEITEN
APPAREIL DE COLLECTE DE LIQUIDE

(30) Priority: 21.12.2001 GB 0130894
(43) Date of publication of application: 15.09.2004
(73) Proprietor: Wills, Trevor, Chesterfield, Derbyshire S40 2JW (GB)
(72) Inventor: Wills, Trevor, Chesterfield, Derbyshire S40 2JW (GB)
(74) Representative: Waddington, Richard
(86) International application number: PCT/GB2002/005765
(87) International publication number: WO 2003/055423

(56) References cited:
- FR-A- 2 638 353
- GB-A- 2 170 711
- US-A- 3 721 243
- US-A- 6 012 181

## Description

The present invention relates to fluid collection apparatus and particularly, although not exclusively, relates to apparatus for collecting urine excreted by individuals suffering from urinary incontinence.

Many stroke victims are left with partial brain damage which can often result in urinary incontinence. Urinary incontinence is a source of acute embarrassment, especially in otherwise healthy individuals, and leads to the individual having a very restricted lifestyle. These restrictions are especially relevant in stroke victims because family and friends would often like to take the individual out of their home, for example, on trips, but often find it very difficult to come to terms with the assistance that would be required of them. Hence, the stroke victims become almost completely housebound which results in depression. The ages of stroke victims range from very young to very old, and urinary incontinence puts great strain not only on the stroke victim themselves, but also on their friends and family. Urinary incontinence is also suffered by post-operation sufferers and patients suffering from prostate problems. In addition, many people take water tablets on a daily basis and hence, have to urinate frequently.

There are currently two types of apparatus available for use by urinary incontinent individuals. The first type of apparatus includes a catheter which requires insertion directly in to the urinary tract connected to a bag in which urine is collected. Unfortunately, the catheter causes discomfort to the individual. The second type of apparatus includes a urine collection bag connected to a convene-type device which may be placed over the penis of the individual and which is prone to coming off leading to embarrassment.

One example of a prior art collection device is given in US 3 721 243 which discloses a collection means with inlet, storage means, sensing means for the volume of fluid, and outlet means.

There are many inherent disadvantages common to both types of apparatus currently available. The devices are manually operated and have no safety or feedback features to improve functionality. In addition, they must be emptied manually by the individual. Movement, such as getting in and out of cars, dislodges the tubes connecting the urine collection bag to the catheter or convene, usually without the wearer knowing which can result in leakage. Furthermore, the wearer has no way of knowing when the bag is full with urine and, therefore, runs the risk that overflow and spillage may occur. The urine bag is normally manually emptied via an exhaust tap on the inside of the patient's ankle. If the patient is not that active or has partial brain damage, a second person is required to empty the urine bag by kneeling down and pressing on the exhaust tap.

In many cases, the apparatus has to be fitted by specialist medical staff and in the case of the catheter device, these have to be correctly taped in position in the urinary tract. Due to these disadvantages of the known equipment, the element of confidence and comfort to the wearer are removed. In addition, nursing staff are reluctant to recommend these devices due to these problems.

It is an aim of embodiments of the present invention to address the above problems and to provide an improved fluid collection apparatus in which control is given to the incontinent individual thereby increasing their confidence and resulting in an improved lifestyle.

According to a first aspect of the present invention, there is provided portable fluid collection apparatus for collecting fluid produced by a person, the apparatus comprising inlet means attachable to the person, said inlet means being operable to collect fluid produced by the person, fluid storage means for storing the fluid, conduit means connecting the inlet means with the fluid storage means, sensing means operable to detect fluid inside the fluid storage means, processing means operable to determine the volume of fluid in the fluid storage means based on output from the sensing means and at least one outlet through which fluid may pass.

Preferably, the apparatus comprises pumping means operable to pump fluid from the fluid storage means through the outlet.

The person may be urinary incontinent, and the fluid may comprise urine. Advantageously, the apparatus is portable and, therefore, may be carried or attached to the person thereby improving his or her lifestyle. Preferably, the said apparatus is attachable to underwear worn by the person, for example, underpants.

Preferably, the inlet means comprises a convene or sleeve which may be attachable to the urethral tract of the person. The inlet means may comprise a catheter for insertion into the urethral tract of the person.

Preferably, the inlet means is manufactured out of non-allergenic material, preferably non-allergenic latex.

Preferably, the fluid storage means comprises at least one substantially flexible container, for example, a storage bag. The fluid storage means may comprise attachment means, by which the said fluid storage means may be attached to a leg of the person. Preferably, the attachment means comprises a strap.

Preferably, the sensing means comprises at least one fluid position detection means operable to detect the presence of said fluid at a first position in the fluid storage means. Preferably, the processing means is operable to determine the position of fluid in the fluid storage means, based on output from the fluid detection means.

Preferably, the sensing means comprises first and second fluid position detection means which detection means are operable to detect the presence of said fluid at first and second positions in the fluid storage means, respectively. Preferably, the first and second fluid position detection means comprise switches enclosed within a casing, which casing is secured to a side of the fluid storage means. Preferably, the first and second fluid position detection means comprise reed switches attached at either end of the casing, which is preferably substantially elongate. Preferably, the casing is substantially permeable to the fluid such that the said fluid is able to pass therethrough. Preferably, the level of fluid in the fluid storage means is substantially the same as that within the casing.

Preferably, the sensing means further comprises switch actuating means floating on the surface of the urine within the casing. Preferably, the switch actuating means floats on the surface of the fluid between the first and second detection means depending on the volume of fluid in the fluid storage means. Preferably, the switch actuating means is operable to induce the first and second detection means to produce an output signal preferably as the said switch actuating means approaches the said detection means. Preferably, the switch actuating means comprises a magnet.

The sensing means may comprise fluid resistance detection means, which is adapted to detect the resistance of the fluid in the fluid storage means. The volume of fluid contained within the storage means may be determined by the processing means using the value of resistance of fluid.

Preferably, the processing means is operable to determine the position of fluid in the fluid storage means, based on output from the first and/or second fluid detection means.

Preferably, the first detection means is located at 50-95% of the total volume of the fluid storage means, more preferably, 70-80% of the total volume of the fluid storage means.

The apparatus comprises signalling means operable to alert the person when the fluid reaches the first position in the fluid storage means based on output from the sensing means. The signalling means may generate audio, visual or vibrating alarm signals. Advantageously, the person is warned by the signalling means when the fluid storage means is nearly full with fluid, thereby allowing the person to empty fluid via the outlet. Advantageously, this reduces the likelihood of overflowing occurring.

Preferably, the processing means actuates the signalling means after receiving a pre-determined number of output signals from the first fluid position detection means. Disdvantageously, turbulence caused by movement of the fluid storage means, for example, when the person moves, may cause the fluid detection means to produce output signals falsely. Therefore, advantageously, the pre-determined number of output signals required by the processing means should minimise the likelihood of false signals resulting in triggering of the signalling means falsely.

Preferably, the apparatus comprises at least two outlets. Preferably, first and second outlets are connected to the fluid storage means by conduit means. Preferably, the first and second outlets comprise first and second valves, respectively. Preferably, the first and second valves are operable to control fluid flow therethrough.

Preferably, the pumping means comprises at least one tube extending into the fluid storage means and arranged so that fluid may be pumped through the said tube and out of the fluid storage means upon actuation of the said pumping means. Preferably, the first outlet is attached to the pumping means. Preferably, the first outlet is located substantially at the top of the fluid storage means. Preferably, the conduit means connected to the first outlet is substantially elongate such that it may be attached to the waist of the person. Preferably, the apparatus further comprises first actuating means by which the person may actuate the pumping means. Preferably, the first actuating means comprises a button or switch located at the waist of the person. Advantageously, the person may empty the fluid storage means via the first outlet by the pumping means.

Preferably, the second fluid position detection means generates a signal when the fluid storage means is substantially empty whereupon the processing means induces the pumping means to stop and, preferably the first valve to close.

Preferably, the second outlet is located substantially at the bottom of the fluid storage means. Preferably, the conduit means connected to the second outlet is arranged to be attached to the ankle of the person. Advantageously, and preferably, the person may empty the fluid storage means via the second outlet by opening the second valve by a second actuating means which is preferably located at the waist of the person.

Preferably, the second fluid position detection means generates a signal when the fluid storage means is substantially empty whereupon the processing means induces the second valve to close.

Preferably, the conduit means comprises tubing, preferably latex or silicone tubing. Preferably, the conduit means is substantially flexible. Preferably, the conduit means comprises expansion means operable to allow expansion along a longitudinal axis thereof. The expansion means may comprise a moulded concertina section operable to expand along its longitudinal axis thereby allowing the individual to move relatively freely without the conduit means becoming disconnected from the inlet means and/or the fluid storage means. Preferably, a plurality of expansion means are spaced along the conduit means. Advantageously, the expansion means reduce the likelihood of fluid leakage occurring from the conduit means.

Preferably, the conduit means comprises means operable to monitor connectivity therealong the conduit means and at junctions, between the inlet means, fluid storage means and/or the outlet. Preferably, said means generates an output detectable by the processing means thereby enabling the detection of a discontinuity or break in the circuit of the conduit means. The conduit means may be etched with conducting lines thereby forming a circuit, for example, copper wire, which send an output to the processing means. Advantageously, if the conduit means become detached from either the inlet means or the fluid storage means, the processing means detects a discontinuity in the circuit.

Preferably, the conduit means comprises means for measuring the flow of fluid therethrough, preferably by at least one flow meter. Preferably, the at least one flow meter is positioned inline with fluid flow through the conduit means. Preferably, the flow meter generates an output detectable by the processing means by which, together with the dimensions of the conduit means, the said processing means is able to calculate the volume of fluid flow therethrough.

The processing means comprises a microprocessor preferably a computer which comprises data storage means. Preferably, the processing means comprises a battery powered ST7-series Flash RAM microprocessor which comprises an 8-bit MCU with single voltage flash memory, ADC, 16-bit timers and interfaces.

To illustrate a way of using the present invention, there is provided an exemplary method of collecting fluid, said method comprising the steps of fitting the apparatus defined in the first aspect to a person.

This may be used when the person is urinary incontinent.

All the features described herein may be combined with any of the above aspects, in any combination that is within the scope of the appended claims.

For a better understanding of the invention, and to show how embodiments of the same may be carried into effect, reference will be made, by way of example, to the accompanying drawings, in which:-
Figure 1 shows a schematic view of a first embodiment of fluid collection apparatus in use;
Figure 2 shows a schematic view showing components of the fluid collection apparatus;
Figure 3 shows an enlarged view of a component of the fluid collection apparatus;
Figure 4 shows a schematic view of an electrical circuit which controls the fluid collection apparatus;
Figure 5 shows a schematic view of a micro-controller; and
Figure 6 shows a schematic view of a second embodiment of the fluid collection apparatus.

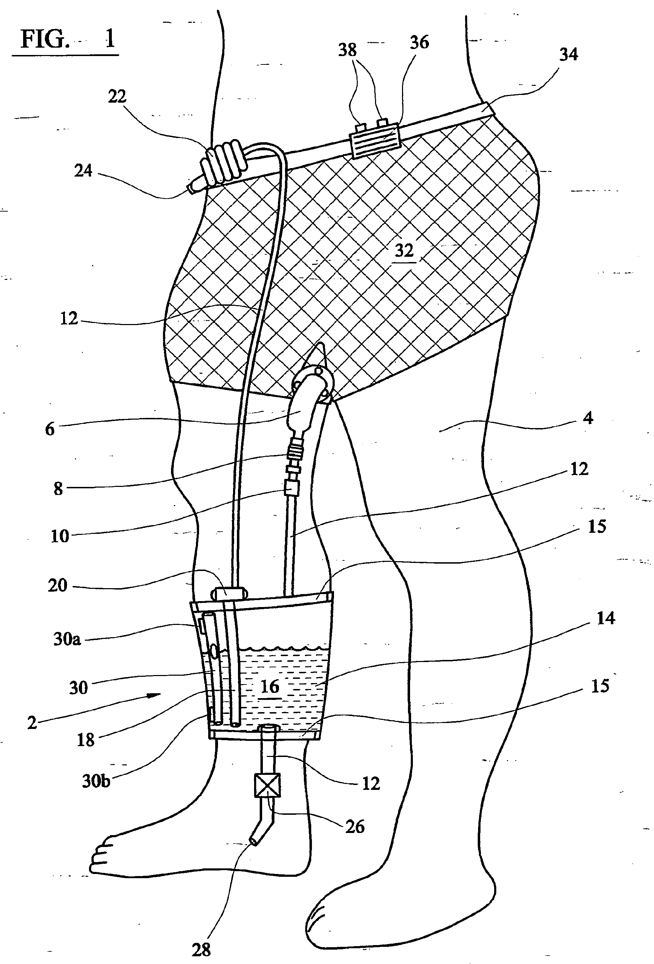

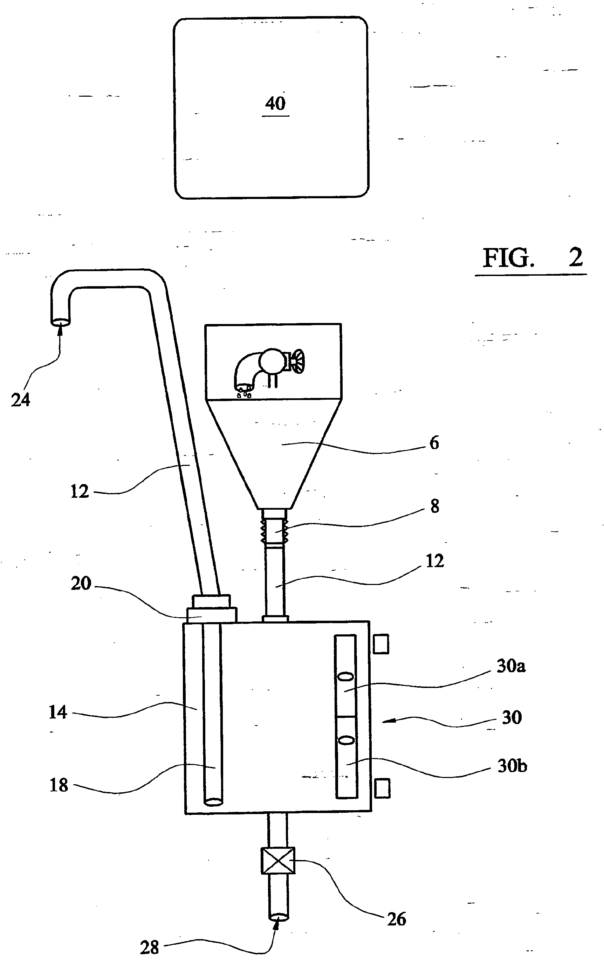

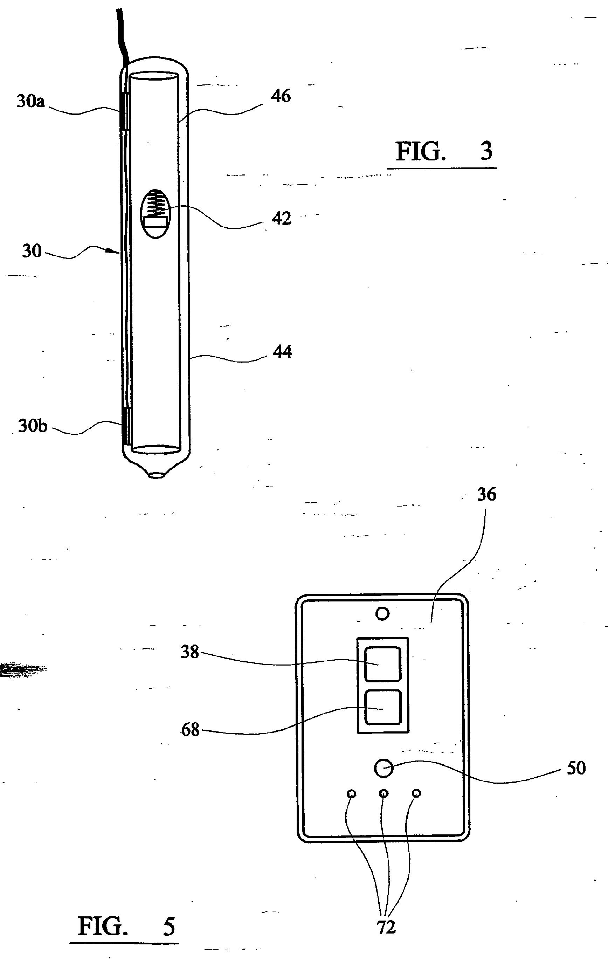

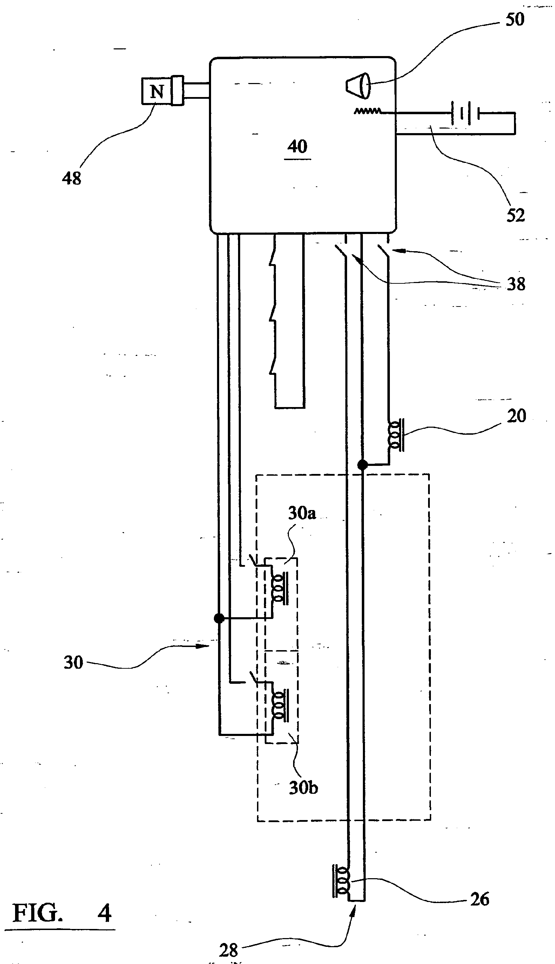

Referring to Figures 1 and 2, there is shown a first embodiment of a fluid collection apparatus 2 fitted to an individual 4 who suffers from urinary incontinence. The apparatus 2 consists of a sleeve or convene 6 which is attached to, or integral with, a pair underpants 32 worn by the incontinent individual 4. The individual's penis is inserted into the sleeve 6 which is made of non-allergenic latex, and arranged such that urine 16 may be collected therein.

Urine 16 excreted by the individual 4 is collected by the sleeve 6 and stored inside a urine bag or container 14 which is attached by straps 15 to one of the legs of the incontinent individual 4. The sleeve 6 is connected to the urine bag 14 by latex or silicone tubing 12 via an expanding section 8 which may be in the form of latex or silicone moulded concertina tubing. The expanding section 8 allows longitudinal expansion thereby allowing the individual 4 to move about relatively freely without the sleeve 6 becoming dislodged from the tubing 12. The tubing 12 is etched with conducting lines along its length to provide flexibility and to enable any disconnections to be detected.

A miniature in-line flow meter 10 is located inside the tubing 12 and measures the volume of urine which passes therethrough into the urine bag 14. The flow meter 10 creates pulses depending upon the flow of urine 16 and enables the calculation of the volume of urine 16 that passes therethrough.

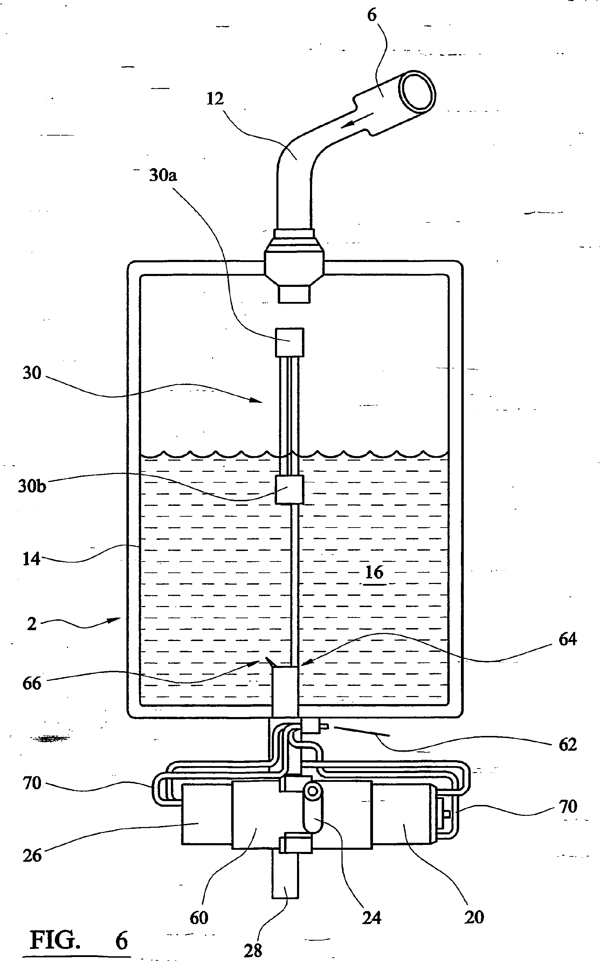

The urine bag 14 has a fixed volume of about 500-800ml and must be emptied on a periodical basis to avoid overflowing. The urine bag 14 is fitted with a urine level detector 30 which is shown in more detail in Figure 3. The apparatus 2 further includes a battery-powered micro-processor/micro-controller 36, which is shown in more detail in Figure 5. The micro-controller 36 consists of a printed circuit board 40, and is attached to the belt of the underpants 32. An ST7 series Flash RAM microprocessor 36 is used featuring an 8-bit MCU with single voltage flash memory, ADC, 16-bit timers and interfaces. The printed circuit board 40 houses the ST7 and electronics for controlling a pump 20 and remote electronic valves 22,26 both of which are activated as is described hereinafter.

Software coding used by the microprocessor 36 is done via a desktop computer (not shown) and a test rig enabling the Flash RAM to be activated and transferred via an in-circuit module.

Referring to Figure 3, there is shown a detailed view of the fluid level detector 30. It consists of an upper sensor 30a and a lower sensor 30b both of which detect the level of urine 16 within the urine bag 14. The fluid level detector 30 consists of a perimeter tube 46 made of solid plastic contained within heat-shrink tubing 44. A floating magnet 42 is contained with the fluid level detector 30 and floats on the surface of the urine 16 as the urine bag 14 fills up. As the volume of urine 16 within the urine bag 14 increases, the floating magnet 42 rises up the tube 46. As the floating magnet 42 reaches the level of the upper reed switch 30a, which is set at 75% of the capacity volume of the urine bag 14, a signal is sent to the microprocessor 36 thereby triggering a warning alarm.

It should be appreciated that other types of fluid level detector 30 may be used. For example, a cradle-type detector (not shown) which resembles a ball-cock housing a small magnet and a hall-effect transistor could be used instead. Alternatively, the fluid level detector 30 may detect differences in the resistance of the urine 16 in the urine bag 14 as shown in Figure 6 and discussed below.

Referring to Figures 1 and 2, urine 16 may be removed from the urine bag 14 through either an upper outlet 24 via an upper valve 22 which is positioned at the waist level of the individual 4, or through a lower outlet 28 via a lower valve 26 which is positioned towards the ankle of the individual 4, depending on the preference of the individual 4. The urine may be pumped out of the urine bag 14 via a tube 18 by pump 20 which is connected by silicone tubing 12 to outlet 24.

Referring to Figure 4, there is shown a circuit diagram of the fluid collection apparatus 2. The microprocessor 36 is provided on a computer board 40 and is battery operated. A battery level sensor 52 is provided to monitor the amount of charge remaining in the battery. The apparatus 2 has two mechanisms for informing the wearer 4 when the urine bag 14 requires emptying, i.e. when the level of urine 16 has reached the level of the upper sensor 30a. The apparatus has a vibrator 48 which is placed close to the skin and which may be set so that it gently vibrates when triggered. The apparatus 2 also has an audio alarm 50 which transmits an alarm signal when triggered. Upon feeling vibrations from the vibrator alarm 48 or hearing the alarm signal from the audio alarm 50, the individual 4 then knows that the urine bag 14 is nearly full and requires emptying. The apparatus 2 has a pump 20 which is configured so that urine 16 may be pumped out of the upper outlet 24. The apparatus 2 as shown in Figure 4, has manual switches 38 which are positioned at waist level and which may be activated to either trigger the pump 20 to pump urine 16 out of outlet 24, or to open valve 26 to allow urine 16 to pass 28 out of outlet 28.

When the urine bag 14 is nearly full, as indicated by the upper sensor 30a, the processor 36 sends out either an audio warning or a silent vibrating signal. The processor 36 interfaces with the pump 20, remote valves 22,26 and level sensors 30a,30b via flexible wires and with the connectivity sensors via the etched tubing and short wires. Once the alert has been received, the individual has a choice on how to empty the urine bag 14. Two manual switches 38 may be employed, one for the pump 20 to empty via the upper outlet 24, and one to open the valve 26 for the lower outlet 28. To empty from the lower outlet 28, the appropriate manual switch 38 is activated which interrupts the microprocessor 36 and in turn opens the valve 26 allowing the urine 16 to leave the urine bag 14. At this time, the microprocessor 36 polls the lower sensor 30b and, when the bag 14 is empty, closes the valve 26. To empty from the upper outlet 24, the appropriate manual switch 38 is activated which interrupts the microprocessor 36 thereby turning on the pump 20, thus extinguishing the urine 16 from the top. The microprocessor 36 polls the lower sensor 30b and when the urine bag 14 is empty, the pump 20 is stopped.

Referring to Figures 5 and 6, there is shown a second embodiment of the fluid collection apparatus 2. Figure 5 shows the micro-controller/processor 36 having a pump switch 38 for controlling the pump 20, and a valve switch 68 for controlling the lower valve 26. Light Emitting Diodes 72 are provided on the controller 36 to inform the user 4 that it is functioning correctly.

Figure 6 shows the lower valve 26, pump 20 and level detector 30 as one integrated unit contained within a housing 60. The level detector has an upper switch 30a and a lower switch 30b, which in the second embodiment measure resistance caused by the urine 16 in the bag 14. The processor 36 determines the volume of urine 16 in the bag 14 and triggers the vibrator 48 or alarm 50 in the usual manner when the urine 16 level gets to a pre-determined height.

The second embodiment has an inlet 66 leading in from the leg bag fluid container 14 via a low level detector 66, and two outlets, i.e. the upper outlet 24 and the lower outlet 28, and associated connecting tubing 70.

The advantages of the urine collection apparatus 2 are manifold. It provides a complete solution enabling patients to leave the confines of their home with confidence. The apparatus 2 has many safety features which are made possible due to the microprocessor 36 continuously checking various parameters. The flow rate of urine 16 into the urine bag 14 is monitored by the flow meter 10, the connectivity of the all of the components and particularly the tubing 12 is continuously checked, and the amount of urine 16 in the bag 14 is monitored by the level sensor 30. The device is portable and comfortable to wear, it minimises the amount of care needed, the wearer does not need to be restrained in his or her movements because of the expanding section 8, and two methods of emptying the bag 14 are provided.

## Claims

1. Portable fluid collection apparatus 2 for collecting fluid produced by a person, the apparatus comprising inlet means 6 attachable to the person, said inlet means 6 being operable to collect fluid produced by the person, fluid storage means 14 for storing the fluid, conduit means 12 connecting the inlet means 6 with the fluid storage means 14, sensing means 30 operable to detect fluid inside the fluid storage means 14, and at least one outlet 24,28 through which fluid may pass, **characterised in that** said apparatus further comprises processing means and signalling means, the processing means, comprising a microprocessor, being operable to determine the volume of fluid in the fluid storage means 14 based on output from the sensing means 30, the signalling means being operable to alert the person when the fluid reaches a predetermined level, wherein the processing means is operable to activate the signalling means.

2. Portable fluid collection apparatus according to claim 1, wherein the apparatus comprises pumping means 20 operable to pump fluid from the fluid storage means 14 through the outlet 24,28.

3. Portable fluid collection apparatus according to any preceding claim, wherein the sensing means 30 comprises at least one fluid position detection means operable to detect the presence of said fluid at a first position in the fluid storage means 14.

4. Portable fluid collection apparatus according to claim 3, wherein the processing means 36 is operable to determine the position of fluid in the fluid storage means 14, based on output from the fluid detection means.

5. Portable fluid collection apparatus according to any preceding claim, wherein the sensing means 30 comprises first and second fluid position detection means 30a,30b which detection means are operable to detect the presence of said fluid at first and second positions in the fluid storage means 14, respectively.

6. Portable fluid collection apparatus according to claim 5, wherein the first and second fluid position detection means 30a,30b comprise switches 38 enclosed within a casing, which casing is secured to a side of the fluid storage means 14.

7. Portable fluid collection apparatus according to any preceding claim, wherein the signalling means 48, 50 is operable to alert the person when the fluid reaches the first position in the fluid storage means 14 based on output from the sensing means 30.

8. Portable fluid collection apparatus according to anyone of the preceding claims, wherein the conduit means 12 comprises means operable to monitor connectivity therealong and at junctions between the inlet means 6, fluid storage means 14 and/or the outlet 24,28, and said means generates an output detectable by the processing means 36 thereby enabling the detection of a discontinuity or break in the circuit of the conduit means 12.

9. Portable fluid collection apparatus according to any preceding claim, wherein the conduit means 12 comprises means for measuring the flow of fluid therethrough, by at least one flow meter 10.

10. Portable fluid collection apparatus according to claim 9, wherein the at least one flow meter 10 is positioned inline with fluid flow through the conduit means 12.

## Patentansprüche

1. Tragbares Fluidsammelgerät 2 zum Sammeln von Fluid, das durch eine Person produziert wird, wobei das Gerät eine an der Person anbringbare Einlasseinrichtung 6 umfasst, welche Einlasseinrichtung 6 dazu ausgelegt ist, durch die Person produziertes Fluid zu sammeln, eine Fluidspeichereinrichtung 14 zum Speichern des Fluids, eine Leitungseinrichtung 12, die die Einlasseinrichtung 6 mit der Fluidspeichereinrichtung 14 verbindet, eine Sensoreinrichtung 30, die dazu ausgelegt ist, Fluid innerhalb der Fluidspeichereinrichtung 14 zu erfassen sowie wenigstens einen Auslass 24, 28 durch den Fluid hindurch gelangen kann, **dadurch gekennzeichnet, dass** das Gerät ferner eine Verarbeitungseinrichtung und eine Signaleinrichtung umfasst, wobei die Verarbeitungseinrichtung einen Mikroprozessor umfasst, der dazu ausgelegt ist, das Volumen von Fluid in der Fluidspeichereinrichtung 14 basierend auf einer Ausgabe von der Sensoreinrichtung 30 zu bestimmen, wobei ferner die Signaleinrichtung dazu ausgelegt ist, die Person zu warnen, wenn das Fluid einen vorbestimmten Pegel erreicht, und wobei die Verarbeitungseinrichtung dazu ausgelegt ist, die Signaleinrichtung zu aktivieren.

2. Tragbares Fluidsammelgerät nach Anspruch 1, wobei das Gerät eine Pumpeinrichtung 20 umfasst, die dazu ausgelegt ist, Fluid von der Fluidspeichereinrichtung 14 durch den Auslass 24, 28 zu pumpen.

3. Tragbares Fluidsammelgerät nach einem vorhergehenden Anspruch, wobei die Sensoreinrichtung 30 wenigstens eine Fluidpositionerfassungseinrichtung umfasst, die dazu ausgelegt ist, das Vorhandensein des Fluids an einer ersten Position in der Fluidspeichereinrichtung 14 zu erfassen.

4. Tragbares Fluidsammelgerät nach Anspruch 3, wobei die Verarbeitungseinrichtung 36 dazu ausgelegt ist, die Position von Fluid in der Fluidspeichereinrichtung 14 basierend auf einer Ausgabe von der Fluiderfassungseinrichtung zu bestimmen.

5. Tragbares Fluidsammelgerät nach einem vorhergehenden Anspruch, wobei die Sensoreinrichtung 30 eine erste und eine zweite Fluidpositionserfassungseinrichtung 30a, 30b umfasst, welche Erfassungseinrichtungen dazu ausgelegt sind, das Vorhandensein des Fluids an einer ersten bzw. an einer zweiten Position in der Fluidspeichereinrichtung 14 zu erfassen.

6. Tragbares Fluidsammelgerät nach Anspruch 5, wobei die erste und die zweite Fluidpositionserfassungseinrichtung 30a, 30b Schalter 38 umfassen, die innerhalb eines Gehäuses eingeschlossen sind, welches Gehäuse an einer Seite der Fluidspeichereinrichtung 14 angebracht ist.

7. Tragbares Fluidsammelgerät nach einem der vorhergehenden Ansprüche, wobei die Signaleinrichtung 48, 50, dazu ausgelegt ist, basierend auf einer Ausgabe von der Sensoreinrichtung 30 die Person zu warnen, wenn das Fluid die erste Position in der Fluidspeichereinrichtung 14 erreicht.

8. Tragbares Fluidsammelgerät nach einem der vorhergehenden Ansprüche, wobei die Leitungseinrichtung 12 eine Einrichtung umfasst, die dazu ausgelegt ist, den Verbindungszustand entlang derselben sowie an Verbindungen zwischen der Einlasseinrichtung 6, der Fluidspeichereinrichtung 14 und/oder dem Auslass 24, 28, zu überwachen, und die Einrichtung eine Ausgabe erzeugt, die von der Verarbeitungseinrichtung 36 erfassbar ist, woduch die Erfassung einer Diskontinuität oder Unterbrechung in der Leitung der Leitungseinrichtung 12 ermöglicht wird.

9. Tragbares Fluidsammelgerät nach einem vorhergehenden Anspruch, wobei die Leitungseinrichtung 12 eine Einrichtung umfasst, um den Fluss von Fluid durch sie hindurch mittels wenigstens eines Durchflussmessers 10 zu messen.

10. Tragbares Fluidsammelgerät nach Anspruch 9, wobei der wenigstens eine Durchflussungsmesser 10 in den Fluidstrom durch die Leitungseinrichtung 12 zwischengeschaltet ist.

## Revendications

1. Appareil portable de collecte de liquide (2) pour la collecte de liquide produit par une personne, l'appareil comprenant un moyen d'entrée (6) pouvant être attaché à la personne, ledit moyen d'entrée (6) étant apte à être actionné pour collecter le liquide produit par la personne, un moyen de stockage de liquide (14) pour stocker le liquide, un moyen de conduit (12) reliant le moyen d'entrée (6) au moyen de stockage de liquide (14), un moyen de détection (30) apte à être actionné pour détecter le liquide à l'intérieur du moyen de stockage de liquide (14) et au moins une sortie (24, 28) au travers de laquelle le liquide peut passer, **caractérisé en ce que** ledit appareil comprend en outre un moyen de traitement et un moyen de signalisation, le moyen de traitement comprenant un microprocesseur, étant apte à être actionné pour déterminer le volume du liquide dans le moyen de stockage de liquide (14) sur la base d'une sortie provenant du moyen de détection (30), le moyen de signalisation étant apte à être actionné pour alerter la personne quand le liquide atteint un niveau prédéterminé, dans lequel le moyen de traitement est apte à être actionné pour activer le moyen de signalisation.

2. Appareil portable de collecte de liquide selon la revendication 1, dans lequel l'appareil comprend un moyen de pompage (20) apte à être actionné pour pomper du liquide à partir du moyen de stockage de liquide (14) au travers de la sortie (24, 28).

3. Appareil portable de collecte de liquide selon l'une quelconque des revendications précédentes, dans lequel le moyen de détection (30) comprend au moins un moyen de détection d'une position du liquide apte à être actionné pour détecter la présence dudit liquide dans une première position dans le moyen de stockage de liquide (14).

4. Appareil portable de collecte de liquide selon la revendication 3, dans lequel le moyen de traitement (36) est apte à être actionné pour déterminer la position du liquide dans le moyen de stockage de liquide (14) sur la base de la sortie provenant du moyen de détection de liquide.

5. Appareil portable de collecte de liquide selon l'une quelconque des revendications précédentes, dans lequel le moyen de détection (30) comprend un premier moyen et un deuxième moyen de détection de position de liquide (30a, 30b), lesquels moyens de détection sont aptes à être actionnés pour détecter la présence dudit liquide respectivement dans une première position et une deuxième position dans le moyen de stockage de liquide (14).

6. Appareil portable de collecte de liquide selon la revendication 5, dans lequel le premier moyen et le deuxième moyen de détection de position de liquide (30a, 30b) comportent des commutateurs 38 enfermés dans un boîtier, lequel boîtier est fixé à un côté du moyen de stockage de liquide (14).

7. Appareil portable de collecte de liquide selon l'une quelconque des revendications précédentes, dans lequel le moyen de signalisation (48, 50) est apte à être actionné pour alerter la personne quand le liquide atteint la première position dans le moyen de stockage de liquide (14) sur la base de la sortie provenant du moyen de détection (30).

8. Appareil portable de collecte de liquide selon l'une quelconque des revendications précédentes, dans lequel le moyen de conduit (12) comprend un moyen apte à être actionné pour surveiller une connexion le long de celui-ci et à des jonctions entre le moyen d'entrée (6), le moyen de stockage de liquide (14) et/ou la sortie (24, 28), et ledit moyen génère une sortie apte à être détectée par le moyen de traitement (36) permettant ainsi la détection d'une discontinuité ou rupture dans le circuit du moyen de conduit (12).

9. Appareil portable de collecte de liquide selon l'une quelconque des revendications précédentes, dans lequel le moyen de conduit (12) comprend un moyen pour mesurer l'écoulement de liquide à travers celui-ci, par au moins un débitmètre (10).

10. Appareil portable de collecte de liquide selon la revendication 9, dans lequel le au moins un débitmètre (10) est positionné en ligne avec l'écoulement de liquide au travers du moyen de conduit (12).
